# EUROPEAN PATENT SPECIFICATION

(11) **EP 3 397 221 B1**
(45) Date of publication and mention of the grant of the patent: **24.06.2020**
(21) Application number: 16815911.9
(22) Date of filing: 07.12.2016
(51) Int. Cl.: A61F 13/08

(54) **COMPRESSION STOCKING**
KOMPRESSIONSSTRUMPF
BAS DE CONTENTION

(30) Priority: 02.01.2016 GB 201600044; 26.08.2016 GB 201614561
(43) Date of publication of application: 07.11.2018
(73) Proprietor: Rosy Ideas Ltd., Chester CH4 9PX (GB)
(72) Inventor: LISTER, Robert Keighley, Chester CH3 6PG (GB)
(74) Representative: Bartle Read
(86) International application number: PCT/GB2016/053845
(87) International publication number: WO 2017/115067

(56) References cited:
- WO-A1-02/34179
- WO-A1-91/05498
- DE-U1-202013 002 992
- US-B1- 6 216 495

## Description

The present invention relates to compression stockings, and in particular to compression stockings formed from more than one part.

The term "compression stocking", or sometimes "elastic compression stocking" refers to a garment worn around the leg which is elastically stretched by the leg when put onto it and which thus applies compression (pressure) to the leg in use. Compression stockings are typically used for a therapeutic purpose. They are typically stiffer elastically than conventional dress socks or stockings, to create greater pressure on the limb. Compression stockings can be categorised according to the pressure they exert in use. In one categorisation system, Class 1 or light compression stockings generate 1.9 to 2.3 kPa (14 to 17mmHg); Class 2 or medium compression stockings generate 2.4 to 3.2 kPa (18 to 24 mmHg) and Class 3 or high compression stockings generate 3.3 to 4.7 kPa (25 to 35mmHg). Pressures of 6.7 kPa (50mmHg) and above are used in some cases. The pressure generated depends upon the size of the stocking and the size of the leg, and compression stockings need to be suitably sized for the individual, but in quoting pressures here and elsewhere in this document it is to be assumed that the stocking is correctly sized for the user.

The term "leg" is used herein in the colloquial sense to refer to the entire lower limb. The term "lower leg" is used to refer to the region of the leg from the ankle to the knee.

Many people suffer from venous and lymphatic insufficiency. This primarily affects the legs and can lead to varicose eczema, lipodermatosclerosis and venous leg ulceration. When the venous system in the legs is compromised, the resulting gravitational effect leads to venous hypertension. Venous insufficiency is a common condition. It is exacerbated by obesity, and so is becoming increasingly problematic in view of increasing obesity levels in the UK and other developed countries. It is the leading cause of varicose eczema and of leg ulceration.

Graduated compression hosiery has been used for many years to overcome venous hypertension. Typically compression is greatest at the ankles and reduces progressively toward the knees and thighs. The effect is to promote return blood flow up the leg. By wearing graduated compression stockings on a regular basis, patients can reduce the risk of varicose eczema and leg ulceration. Some graduated compression stockings extend from the foot to a region below the knee, while some extend above the knee to the thigh region.

The greater the tension in the stocking the greater the improvement in venous return. However, stockings of greater tension are more difficult to get on and off. Many of the patients requiring compression stockings are elderly, suffer poor mobility and may have arthritic joints, which adds to the difficulty involved. Physical force is needed to draw the stocking up the leg whilst putting it on. During removal there is a tendency for the leg part of a compression stocking to bunch just above the ankle, and moving the bunched material past the ankle requires a degree of force which can be difficult for a user lacking manual strength to exert.

A particular problem is that users of compression stockings may suffer from skin lesions, especially ulcers. Dragging a stocking over an ulcer is potentially painful and injurious.

There are numerous known devices to help the patient to get compression stockings on, but often the patient struggles later in the day to remove the stockings. This can lead to panic and many patients abandon use of compression stockings as a result.

The problems associated with putting on and taking off compression stockings are a major barrier to their use.

EP1337211 (Gloria Maglieria Elastica S.R.L) discloses an elastic stocking which is said to be easier to put on and which is formed in two tubular parts. One of these (we will refer to it as the "foot part") is shown to be in the form of an open-toed sock worn on the foot and extending mid-way up the calf. The other part (we will refer to it as the "leg part") is shown as extending from the ankle to just below the knee, and in a long region of overlap between these parts the compressive force of each individual part is approximately halved, so that in the overlap region the "integrity" of the stocking is maintained. The document does not address the question of how the stocking is to be put onto the leg. However a product of this type is sold by Gloria Med S.p.A under the trade name UlcerKit. Instructions offered in relation to that product show it being put on by first putting on the leg part and then putting on the foot part, drawing that up to surround the leg part where they overlap. Other than the stocking being in two parts, no other aspect of their invention facilitates the donning or removal of the stocking.

Putting the compression stocking on is still subject to problems. Consider in particular the use of such a compression stocking on a leg affected by a venous ulcer. These are most often found at or just above the ankle. In this case putting on the two part stocking may involve dragging the entire leg part over the ulcer, which is highly undesirable. Another problem is that it may be difficult for the user to judge how far up the leg the leg part of the compression stocking needs to be positioned, in order for it to overlap to the desired extent with the foot part. This may necessitate adjustment of the position of the leg part after the foot part is put on, but that adds to the inconvenience of the operation and may be problematic for users with limited strength or manual dexterity.

There are other known approaches to the problem of how to facilitate putting on a compression stocking. Some involve the use of zips or other fasteners, but these can create discomfort and are potentially unsightly.

At least one commercially available product uses a relatively low compression inner stocking and a higher compression outer stocking, each of them being shaped to cover the foot and lower leg. This product provides higher compression used to promote healing of established ulcers, rather than being used for the treatment of venous insufficiency. The inner stocking in one commercially available product of this type contributes compression of 1.3kPa (10mmHg) and the outer stocking contributes 4kPa (30 mmHg). The inner stocking is to be put on first and has a slippery outer surface to reduce friction. But both stockings remain in place in use, and this is not optimal from the point of view of comfort. The two layers are also bulky to wear and less cosmetically acceptable than a single layer stocking.

WO91/05498 discloses a more elaborate arrangement in which an inner socklet used to facilitate donning of a compression stocking is to be pulled out of an open toe of the stocking once it is in place. This system is only valid for use with open toe stockings. Many patients find that open toe stockings are uncomfortable. Also this system offers no easy facility for removing the stocking once on the leg.

So despite the widespread and long standing adoption of compression stockings and the long standing recognition of the problems explained above, a need remains for an improved compression stocking which is convenient to put on and to take off. Desirably it should, in terms of its cosmetic appearance and of the compression it provides, be broadly comparable to a conventional unitary compression stocking. It is also desirable that the compression stocking should be capable of economical manufacture.

According to a first aspect of the present invention there is a compression stocking comprising a first part comprising:
a foot portion configured to be worn on the foot;
an ankle portion configured to extend above the malleolus in use; and
a first overlap portion above the ankle portion, the first overlap portion being formed to exert in use a pressure which is reduced compared to a pressure exerted by the ankle portion, and a second part which is tubular and comprises
a second overlap portion and
a leg portion which extends in use up the leg from the overlap portion to surround at least part of the lower leg,
the compression stocking being characterised in that the second overlap portion is able to be placed around the first overlap portion, the compression stocking providing a graduated pressure profile,
in that an outer surface of the first part and an inner surface of the second part are formed in a manner which provides a low coefficient of friction between them, enabling the compression stocking to be put on by putting the first part on first, and then drawing the second part over the first part and pulling it up the leg to bring the second overlap portion into position around the first overlap portion, so that passage of the second part over the foot and ankle is facilitated by the first part,
and in that the first part is provided with a visible external marking, exposure of which indicates when the second part has been drawn to its required position.

Adapting the compression stocking to be put on in this manner is counterintuitive. It is highly desirable that there should be little or no slip of the overlap portions of the two parts relative to one another, since if the overlap portions are not positioned one within the other then the pressures they exert will not sum correctly to provide the desired graduated compression profile. From this point of view it may be regarded as desirable to provide high friction engagement between the two parts of the compression stocking, making it difficult to drag the second part over the first.

But the inventor has recognised that the present invention can be used to provide a compression stocking that is both easier to put on and take off, and highly capable of resisting slippage.

The invention provides various advantages. Where the compression stocking is used to treat a venous ulcer, for example, the first part can cover the region of the ankle in which the ulcer is found, and serves to cover and protect the ulcer as the second part is then drawn into position. The coefficient of friction between the second part and the first part may be lower than that between the second part and the foot/ankle itself, meaning that the force needed to draw the second part into position is reduced, which is highly desirable for users with limited strength. And there is no need for trial and error in positioning the second part upon the leg because when it is put on, the first part is already in position to serve as a guide in this respect.

In accordance with a second aspect of the present invention there is a method of putting on a compression stocking comprising forming the compression stocking in at least two parts, a first part of the compression stocking comprising
a foot portion configured to be worn on the foot;
an ankle portion configured to extend above the malleolus in use;
a first overlap portion above the ankle portion, the first overlap portion being formed to exert in use a pressure which is reduced compared to a pressure exerted by the ankle portion; and
a visible external marking;
and a second part of the compression stocking being tubular and comprising
a second overlap portion and
a leg portion which extends in use up the leg from the overlap portion to surround at least part of the lower leg,
the method being characterised in that it comprises putting the first part on first, and then drawing the second part over the first part and pulling it up the leg to bring the second overlap portion into position around the first overlap portion, so that passage of the second part over the foot and ankle is facilitated by the first part,
and in that the visible external marking is located such that it is revealed when the second part has been pulled far enough up the leg to bring the first and second overlap portions into their intended position with respect to one another.

Specific embodiments of the present invention will now be described, by way of example only, with reference to the accompanying drawings in which:-
Figure 1 shows the medial aspect of two parts forming a compression stocking according to the present invention;
Figure 2 is similar to Figure 1 but shows the two parts positioned as in use;
Figure 3 is an enlarged scrap view, in section, of an overlap region of the compression stocking;
Figure 4 shows the medial aspect of a first part of a further compression stocking embodying the present invention;
Figure 5 is a side view of still a further compression stocking embodying the present invention;
Figure 6 is a side view of yet a further compression stocking embodying the present invention; and
Figure 7 is a view from the rear of yet another compression stocking embodying the present invention.

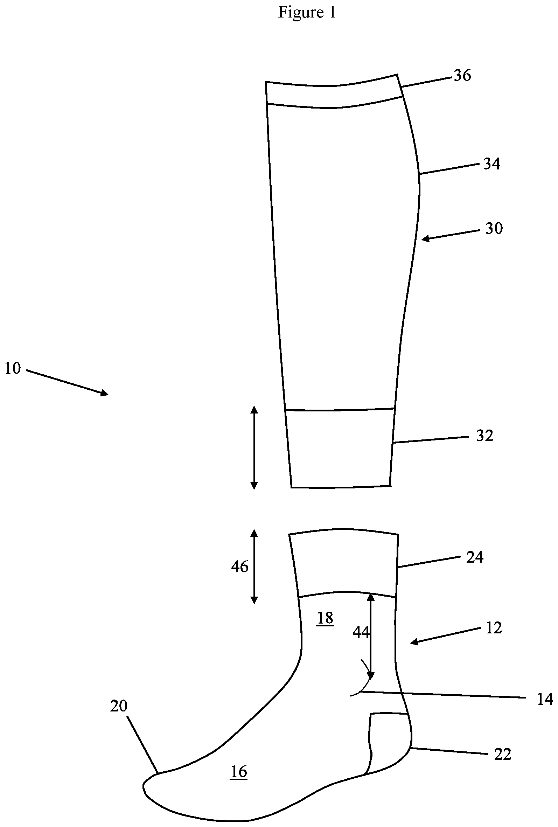

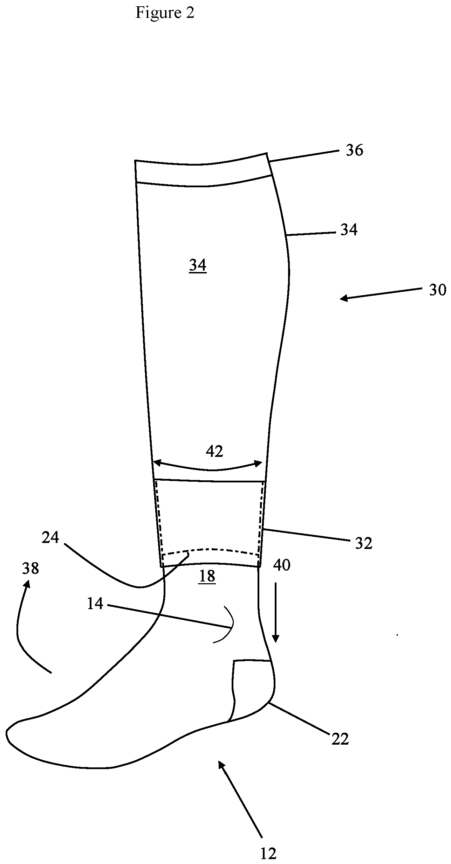

Compression stocking 10 illustrated in Figures 1 and 2 is formed in two separate parts, in order to facilitate putting it on and taking it off.

A first part 12 is shaped and sized in the manner of a sock to be worn upon the foot of a user and to extend above the malleolus, whose profile is indicated at 14. The first part 12 has a foot portion 16 and an ankle portion 18. In the present example the foot portion 16 incorporates a closed toe region 20. It is also shaped to provide a bulbous heel region 22. The ankle portion is tubular and leads continuously to a tubular first overlap portion 24.

A second part 30 of the compression stocking is separately formed from the first and is of tubular form, having at its lower end (terms such as "upper", "lower" and "vertical" are to be understood, throughout this description and the appended claims, to be used with reference to the orientation of the compression stocking when it is worn by a standing user) a second overlap portion 32, above which is a leg portion 34. In the present example the leg portion is to surround the shin and calf, extending to a level just below the user's knee. At its upper edge it carries a high friction welt 36 to resist any tendency for the compression stocking to move down the leg during physical activity.

When the compression stocking 10 is correctly worn on the leg (Figure 2), the first overlap portion 24 is within the second overlap portion 32.

The illustrated compression stocking 10 is a graduated stocking. The pressure it exerts decreases in a direction up the leg, in order to urge blood upwardly and assist venous return flow. Discontinuities in the pressure profile are undesirable since they may impair this function. In the region 24/32 where the first and second parts of the stocking overlap, the pressures they each contribute due to their elasticity are added, so that if these regions of the stocking parts had the same elastic properties as the remainder of the compression stocking 10 then pressure in this overlap region could be excessive. To avoid this, one or both of the overlap portions 24 and 32 contribute a reduced pressure in comparison to the adjacent regions of the compression stocking 10, so that the total pressure exerted by the overlap portions 24 and 32 *together* is approximately equal to the pressures exerted by those adjacent regions. This pressure reduction may be because the material of the overlap region(s) is less stiff than the neighbouring material and/or because the overlap regions are, when unstressed, larger in circumference than the neighbouring regions, so that they are stretched less when on the leg.

In the present embodiment, the first overlap portion is elastically less stiff, in the circumferential direction, than the neighbouring portion of the ankle region 18 of the first part 12 of the stocking. The second overlap portion 32 is elastically less stiff, in the circumferential direction, than the neighbouring portion of the calf region 18 of the second part 30 of the stocking. In the present embodiment each of the overlap portions 24, 32 contributes approximately half of the total pressure in the overlap region. This proportion may be different in other embodiments. In some embodiments the pressure exerted by the overlap portion 24 of the first part 12 is larger than the overlap portion 32 of the second part 30, in order to increase friction between the overlap portion 24 and the skin beneath.

The illustrated stocking is manufactured by knitting and the knitted fabric is thinner and less stiff in the overlap regions 24, 32. This is advantageous in that the two layers together in the overlap region are not excessively thick or bulky, but instead are comparable in thickness to the adjacent regions of the stocking above and below.

The result is that the pressure exerted on the leg varies generally in the desired, graduated, manner along the length of the leg, without a dramatic discontinuity at the overlap region 24/32. The sum of the pressures contributed by the two overlap parts 24, 32 where they overlap is roughly equal, at the lower end of this overlap region, to the pressure exerted by adjacent material of the ankle portion 18 alone, and is roughly equal, at the upper end of the overlap region to the pressure exerted by adjacent material of the leg portion 34 alone.

The compression stocking 10 is intended and adapted to be put on in the following manner.

The first part 12 is put on before the second part 30. Drawing the first part 12 onto the foot and ankle is a relatively straightforward process because it extends a limited distance above the ankle, so the length of the material being drawn over the foot is small compared to a one-piece stocking. It is not desirable to place excessive tension on the overlap portion 24, which is of relatively light material, but the positioning of the overlap 24/32 ensures that there is ample material below this portion, and above the ankle, by which the first part 12 can be grasped.

The wearer then flexes the ankle joint a few times (i.e. carries out dorsiflexion and plantar extension) which helps to settle the first part 12 in a stable position on the foot and ankle.

Once the first part 12 is in place, the second part 30 is put on. This involves drawing the second part 30 over the first part 12. During this process the foot and ankle are protected from rubbing by the first part 12. The first part 12 helps the second part 30 to slide over the foot and ankle and into position higher up the leg. The second part 30 slips easily over the first part 12, due to the properties of the fabrics from which they are formed. In addition the first part 12 covers the toes, toenails and other irregularities of the foot, providing a smooth shape for the second part 30 to slip over and preventing catching of the second part 30 on such irregularities. The first part 12 therefore serves two functions. It is not only an inherent part of the compression stocking, but it also serves to facilitate the application of the second part 30.

When removing the compression stocking 10, the second part 30 is removed before the first part 12. The first part 12 again helps the second part 30 to slide over the foot and ankle, protecting the skin. Typically the second portion, rather than sliding over the lower calf and shin, is rolled down over itself, so that by the stage at which it is drawn off the ankle and foot it has been turned inside out.

The parts 12, 30 of the compression stocking 10 are formed in such a manner that the coefficient of friction between surfaces of the stocking as it is put on and taken off is low. When putting on the stocking 10, the inner surface of the second part 30 slips easily over the outer surface of the first part 12. When taking the stocking 10 off, the second part 30 is typically turned back upon itself, and in this process the outer surface of the second part 30 is drawn over itself and over the outer surface of the first part 12, these surfaces again being formed in a manner which enables them easily to slip without need of excessive manual force.

The relevant coefficient of friction between the surfaces of the stocking may be characterised in that it is low enough to enable the second part 30 to be drawn over the first part 12 and into its required position with only moderate manual force. Alternatively the friction may be characterised in that it is not sufficient to cause the first part 12 to be drawn along the leg as the second part 30 is pulled over it.

The surfaces of the parts 12, 30 of the compression stocking 10 may be shiny. They may be silky. They may be smooth. They may be low friction surfaces. They may be gloss, as opposed to matt, surfaces.

The material of the parts 12, 30 of the compression stocking 10 may be manufactured by known tubular knitting techniques. Suitable elastic materials include fibres of natural rubber, spandex, nylon and cotton, and elastane, which may be used in combination. Knitted elastic materials are used in the present embodiment.

As noted above, it is desirable to resist slippage of one overlap portion 24 relative to the other overlap portion 32. The inventor has established that this can be achieved even without any fasteners and despite the low coefficient of friction between the first and second parts 24, 32. Once the compression stocking 10 is correctly worn upon the leg, it is flexure of the joints and muscle activity that tend to cause any slippage. But consider the forms of motion involved. Dorsiflexion - that is, turning the foot about the ankle joint in the direction indicated by arrow 38 in Figure 2 - may be expected to elongate the underside of the first part 12, creating tension acting in the direction of arrow 40 tending to move the rear of the ankle portion downward. But in practice tension in the material along the sole of the foot is reacted through the engagement of the bulbous heel portion 22 with the user's heel. In this way the heel portion 22 acts in the manner of a tether, so that any vertical movement tends to be limited to the region of the arrow 40 above the heel. Activity of the calf muscle produces essentially horizontal movement (changes in leg circumference) as suggested by arrow 42 in Figure 2, but due to its direction this does not tend to cause slippage in the overlap region. Vertical movement of the first part 12 in the area indicated by arrow 40 is resisted by friction between the first part 12 and the skin of the wearer, and not solely by friction between the two parts of the stocking 10.

The position of the overlap region 24/32 is considered to have a bearing on the stability of the overlap 24, 32. The greatest vertical movement is experienced around the area of the Achilles tendon, at the level of the ankle, so if the overlap is too close to the ankle its stability may be impaired, tending to pull the overlap apart. Vertical movement of the compression stocking 10 in this area is found in practice to diminish in a direction upward from the ankle, so that an overlap region high up the lower leg can be expected to be more stable. But if it is too high up the leg then the difficulty of putting on the first part 12 may be increased due to the extra length of this part. Also an overlap high up the lower leg may be regarded as cosmetically unacceptable. In the present embodiment the lower edge of the first overlap portion 24 is sited approximately 6 to 8 centimetres above the summit of the medial malleolus 14. This dimension is indicated by arrow 44 in Figure 1. It results in a first portion of small enough length to be easily put on, and provides stability of the overlap. Also the ankle portion 18 and the first overlap portion 24 are together deep enough to be easily grasped to pull the first part 12 onto the foot. The most critical area for compression in treatment of venous ulcers is the portion of the ankle just above the malleoli, as this is where most venous ulcers occur. The suggested positioning of the overlap region provides a run of continuous material in this region, to ensure good functional compression, the overlap region 24, 32 being above this critical area of the ankle.

The dimension 44 may be varied in other embodiments.

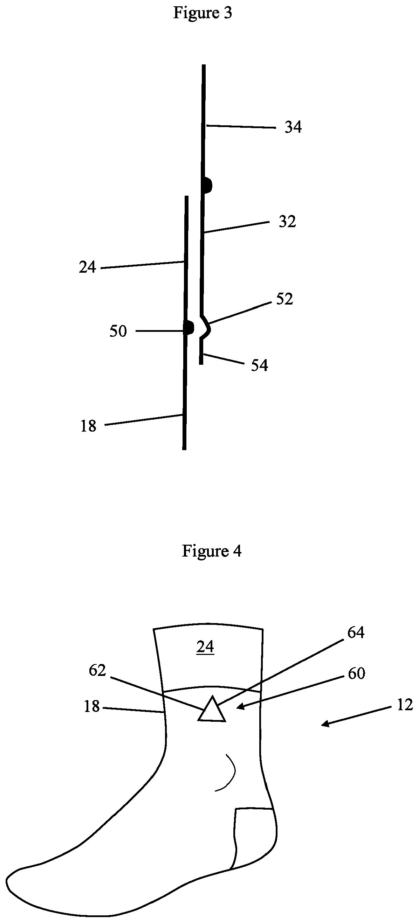

The depth of the overlap zone (arrow 46 in Figure 1) is approximately 5 centimetres in the present embodiment, but could be varied in other embodiments. Figure 3 shows a detail of the overlap in a form which is somewhat stylised. The material forming the overlap is depicted in section and it can be seen that at a transition between the first overlap portion 24 and the ankle portion 18, a ridge 50 is present. In the present embodiment this is a result of the knitting process used to form the compression stocking 10, although it could take other forms. It somewhat deforms the material of the second overlap portion 32 in a region indicated at 52, and tends to resist slippage in the overlap region. Note also that the second overlap portion 32 is slightly larger in depth than the first overlap portion 24, providing a band 54 (see Figure 3) of the second overlap portion 32 which extends onto the ankle portion 18. This helps to ensure, in the event that the overlap should be pulled apart slightly, that there is not a local loss of pressure which could impair the function of the compression stocking 10.

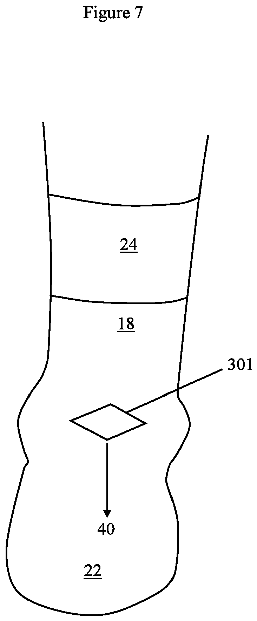

Figure 7 represents an optional development of the compression stocking 10 according to an embodiment of the invention. This includes an area 301 of fabric which is elastically less stiff than the fabric around it. The area 301 is disposed at the rear of the lower leg in use, and more specifically in the present example it is in the region of the mid aspect of the Achilles tendon. The area 301 is able to stretch more easily (i.e. with less tension) than surrounding material along the direction of arrow 40 in response to dorsiflexion, and due to its low stiffness it serves to reduce any resultant tension referred to the first overlap zone 24 tending to move that zone of the stocking 10 along the leg. In this way the area 301 helps to prevent movement of the first overlap zone 24 and stabilise the overlap. The area 301 can be formed by local variation of the weave of the stocking. In Figure 7 the area 301 is depicted as a narrow diamond shape, but other shapes could be used.

When putting the compression stocking 10 on, the second part 30 needs to be pulled far enough up the leg to bring the overlap portions 24, 32 into their proper positions, and no further. To facilitate this the first part 12 may be provided on its outer surface with a marking which is revealed as the second part 30 is drawn up the leg, providing a visual indication when the required position has been reached. This marking may be positioned below the desired level of the lower edge of the second part 30, so that it begins to be revealed somewhat before the desired position is achieved. It can vary over its vertical extent in a manner which enables the user to judge how much further the second part 30 needs to be drawn up the leg to achieve the required position. In the embodiment illustrated in Figure 4, the marking 60 takes the form of a triangle having sides 62, 64 which converge upwardly and which meet just below the lower edge of the first overlap portion 24. When the apex of this triangle is visible, the user knows that the second part 30 is properly positioned. The marking could take a variety of other forms. The marking 60 is in this embodiment disposed on the medial aspect of the leg, which is advantageous both in that (a) it is easily viewed by the wearer as the stocking is put on and (b) being directed toward the other leg, it is visually inconspicuous in use.

The formation of the foot portion 16 with a closed toe 20 is advantageous in that the toes are covered while the second part 30 of the stocking is pulled over them. In this way it is ensured that the toes do not catch on the second part, which facilitates the donning of part 30 and which could additionally or alternatively cause damage to the stocking. Nonetheless the present invention may be implemented with an open toe form of stocking.

In an alternative embodiment of the invention (not illustrated) a light, low compression, low friction foot sock may be provided in addition to the first and second parts 12, 30. This foot sock would be put on before the first part 12, typically extending just above the malleolus. The first part 12 is the put on over the ankle sock, which thus serves to reduce the effort needed to pull on the first part 12 and to prevent catching on the toenails etc. This embodiment may be especially easy for users to put on, and suited to those who struggle most to put on a conventional compression stocking. This embodiment is also well suited to the use of an open-toed first part 12.

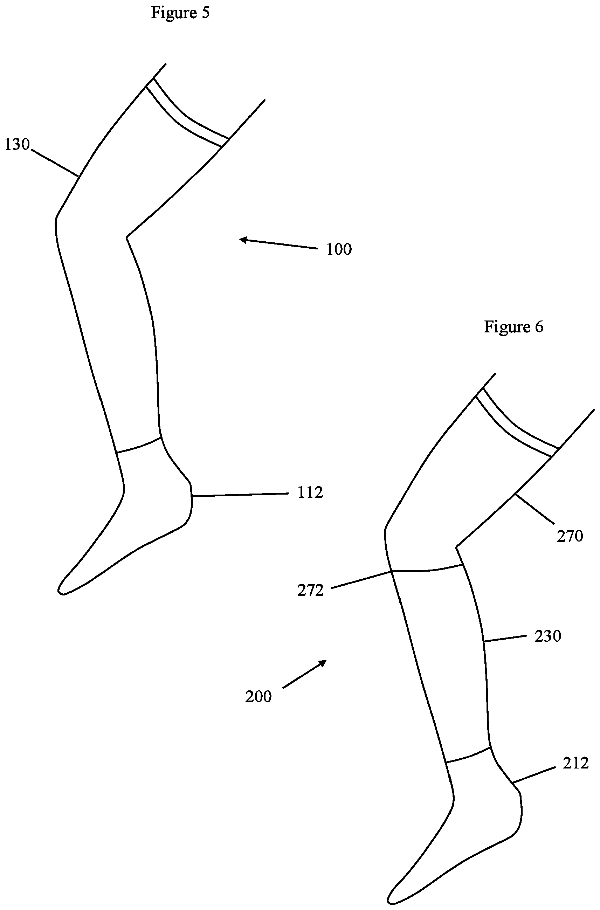

The compression stocking 10 illustrated in Figures 1 and 2 terminates below the knee, but the present invention may be embodied in a compression stocking which extends to the thigh. Such an embodiment 100 is illustrated in Figure 5, this version being once more formed in two parts - a first part 112 and a second part 130, with the overlap between them being a short distance above the ankle. However Figure 6 shows an embodiment in which, to further facilitate putting compression stocking 200 onto the leg, it is broken into first, second and third parts 212, 230, 270. In this case the sequence for putting on the compression stocking 200 involves putting the first part 212 onto the foot and ankle, then putting the second part 230 onto the lower leg, then putting on the third part 270 which extends to the thigh region. The second and third parts 230, 270 overlap in a region 272.

In the illustrated embodiments the material forming the first and second overlap regions 24, 30 is the same as the material of the compression stocking 10, save for the knit pattern that provides a reduced modulus of elasticity in these regions. But in other embodiments the overlap regions 24, 30 may be formed in a manner which increases friction between the overlap regions. This may be achieved by a coating on one or both of the facing surfaces at the overlap, or through changes in the weave of the fabric or its material in these areas.

In still other embodiments the compression provided by the first part 12 to the foot of the wearer may be relatively low, with greater compression being applied by the second part 30 to the wearer's leg.

Individual parts can be manufactured in different sizes, and "made to measure", for different diameters of the various parts of the leg and for different leg lengths. Some patient's legs are of highly abnormal shape and compression stockings need to be fitted based on measurements at the foot, ankle, mid-calf and upper calf. Forming the compression stocking in two parts makes possible different permutations of stock parts to obtain a correctly fitting stocking.

The simple multi-part designs illustrated herein eliminate the need for any bulky joiners or fasteners and therefore preserve the cosmetic acceptability of the garment, which is important for patient compliance.

Compression stockings require laundering, and over time this reduces their elasticity and functionality. An advantage of the illustrated embodiments is that the parts of the compression stocking 10, 100, 200 can be washed separately. The first part 12, 112, 212 is expected to require more frequent laundering because it encases the foot, but it is of less importance in terms of providing limb compression than the second part 30, 130, 230, which can be laundered much less often. This can increase the functional life of the compression stocking.

The compression stocking 10 will typically provide a pressure which, at its greatest, is more than 1.9kPa. The compression stocking 10 may be in any of the compression classes mentioned above with reference to the prior art. That is, it may be:-
light compression - 1.9 to 2.3 kPa (14 to 17mmHg);
medium compression - 2.4 to 3.2 kPa (18 to 24 mmHg)
high compression stockings generate - 3.3 to 4.7 kPa (25 to 35mmHg) or higher.

## Claims

1. A compression stocking (10) comprising a first part (12) comprising:
a foot portion (16) configured to be worn on the foot;
an ankle portion (18) configured to extend above the malleolus in use; and
a first overlap portion (24) above the ankle portion (18), the first overlap portion (24) being formed to exert in use a pressure which is reduced compared to a pressure exerted by the ankle portion (18),
and a second part (30) which is tubular and comprises
a second overlap portion (32) and
a leg portion (34) which extends in use up the leg from the overlap portion to surround at least part of the lower leg,
the compression stocking being **characterised in that** the second overlap portion (32) is able to be placed around the first overlap portion (24), the compression stocking providing a graduated pressure profile,
**in that** an outer surface of the first part (12) and an inner surface of the second part (30) are formed in a manner which provides a low coefficient of friction between them, enabling the compression stocking to be put on by putting the first part (12) on first, and then drawing the second part (30) over the first part (12) and pulling it up the leg to bring the second overlap portion (32) into position around the first overlap portion (34), so that passage of the second part over the foot and ankle is facilitated by the first part,
and **in that** the first part (12) is provided with a visible external marking (60), exposure of which indicates when the second part has been drawn to its required position.

2. A compression stocking as claimed in claim 1 provided with a set of instructions to a user directing the user to put the stocking on by putting the first part (12) on first, and then drawing the second part (30) over the first part (12).

3. A compression stocking as claimed in any preceding claim in which the marking (60) is disposed below the first overlap portion (24) and has a certain vertical extent, the marking (60) changing in a progressive manner over this vertical extent so that as the second part (30) is drawn up the leg the exposed part of the marking gives a basis for judging how much further the second part (30) needs to be drawn to reach its required position.

4. A compression stocking as claimed in claim 3 in which the marking (60) comprises a pair of lines or edges (62, 64) which converge upwardly, and which meet at the level which is to be reached by a lower edge of the second part.

5. A compression stocking as claimed in any preceding claim in which the first part is of closed toe form.

6. A compression stocking as claimed in any preceding claim in which the first overlap portion (24) has a lower edge which is above the malleolus in use.

7. A compression stocking as claimed in claim 6 in which the lower edge of the first overlap portion (24) is between 5 and 10 centimetres above the summit of the medial malleolus in use.

8. A compression stocking as claimed in any preceding claim in which the lower edge of the first overlap portion (24) is between 6 and 8 centimetres above the summit of the medial malleolus in use.

9. A compression stocking as claimed in any preceding claim in which, in use, the first and second overlap portions (24, 32) contribute substantially equal pressures.

10. A compression stocking as claimed in any preceding claim in which the second overlap portion (32) is deeper, in a direction extending along the leg in use, than the first overlap portion (24).

11. A compression stocking as claimed in claim 10 in which the second overlap portion (32) is between 0.5 centimetres and 1.5 centimetres deeper than the first overlap portion (24).

12. A compression stocking as claimed in any preceding claim in which an area of the foot portion (16) in the vicinity of the Achilles heel in use is formed to have a lower elastic stiffness than material surrounding it, and so to stretch preferentially during dorsiflexion.

13. A method of putting on a compression stocking (10), comprising forming the compression stocking in at least two parts, a first part (12) of the compression stocking comprising
a foot portion (16) configured to be worn on the foot;
an ankle portion (18) configured to extend above the malleolus in use;
a first overlap portion (24) above the ankle portion, the first overlap portion (24) being formed to exert in use a pressure which is reduced compared to a pressure exerted by the ankle portion (18); and
a visible external marking;
and a second part (30) of the compression stocking being tubular and comprising
a second overlap portion (32) and
a leg portion (34) which extends in use up the leg from the overlap portion to surround at least part of the lower leg,
the method being **characterised in that** it comprises putting the first part (12) on first, and then drawing the second part (30) over the first part and pulling it up the leg to bring the second overlap portion (32) into position around the first overlap portion (24), so that passage of the second part (30) over the foot and ankle is facilitated by the first part,
and **in that** the visible external marking is located such that it is revealed when the second part has been pulled far enough up the leg to bring the first and second overlap portions into their intended position with respect to one another.

14. A method as claimed in claim 12 further comprising forming the first and second parts (12, 30) in such a manner that friction between them as the second part (30) is drawn over the first part is low enough to enable the second part (30) to be drawn over the first part (12) without drawing the first part (12) along the leg.

## Patentansprüche

1. Kompressionsstrumpf (10), umfassend einen ersten Teil (12), der Folgendes umfasst:
einen Fußabschnitt (16), der konfiguriert ist, um an dem Fuß getragen zu werden;
einen Knöchelabschnitt (18), der konfiguriert ist, um sich in Verwendung über dem Fußknöchel zu erstrecken; und
einen ersten Überlappungsabschnitt (24) über dem Knöchelabschnitt (18), wobei der erste Überlappungsabschnitt (24) ausgebildet ist, um in Verwendung einen Druck auszuüben, der im Vergleich zu einem durch den Knöchelabschnitt (18) ausgeübten Druck reduziert ist,
und einen zweiten Teil (30), der röhrenförmig ist und Folgendes umfasst:
einen zweiten Überlappungsabschnitt (32) und
einen Beinabschnitt (34), der sich in Verwendung aus dem Überlappungsabschnitt an dem Bein hinauf erstreckt, um wenigstens einen Teil des Unterschenkels zu umgeben,
wobei der Kompressionsstrumpf **dadurch gekennzeichnet ist, dass** der zweite Überlappungsabschnitt (32) um den ersten Überlappungsabschnitt (24) herum angebracht werden kann, wobei der Kompressionsstrumpf ein abgestuftes Druckprofil bereitstellt, dadurch, dass eine Außenoberfläche des ersten Teils (12) und eine Innenoberfläche des zweiten Teils (30) in einer Weise ausgebildet sind, die eine niedrige Reibungskoeffiziente zwischen ihnen bereitstellt, wodurch ein Anziehen des Kompressionsstrumpfes durch Anziehen des ersten Teils (12) zuerst und anschließendes Ziehen des zweiten Teils (30) über den ersten Teil (12) und ein Hochziehen an dem Bein hinauf ermöglicht wird, um den zweiten Überlappungsabschnitt (32) um den ersten Überlappungsabschnitt (34) herum in Position zu bringen, so dass ein Durchgang des zweiten Teils über dem Fuß und dem Knöchel durch den ersten Teil vereinfacht wird,
und dadurch, dass der erste Teil (12) mit einer sichtbaren äußeren Markierung (60) versehen ist, deren Freilegen anzeigt, wenn der zweite Teil in seine erforderliche Position gezogen wurde.

2. Kompressionsstrumpf nach Anspruch 1, der mit einer Reihe von Anweisungen an den Benutzer versehen ist, die den Benutzer leiten, den Strumpf durch Anziehen des ersten Teils (12) zuerst und anschließendes Ziehen des zweiten Teils (30) über den ersten Teil (12) anzuziehen.

3. Kompressionsstrumpf nach einem der vorhergehenden Ansprüche, wobei die Markierung (60) unter dem ersten Überlappungsabschnitt (24) angeordnet ist und eine gewisse vertikale Erstreckung aufweist, wobei sich die Markierung (60) über diese vertikale Erstreckung zunehmend verändert, so dass der freiliegende Teil der Markierung beim Ziehen des zweiten Teils (30) an dem Bein hinauf eine Basis zum Beurteilen gibt, wie viel weiter der zweite Teil (30) gezogen werden muss, um seine erforderliche Position zu erreichen.

4. Kompressionsstrumpf nach Anspruch 3, wobei die Markierung (60) ein Paar von Linien oder Rändern (62, 64) umfasst, die nach oben zusammenlaufen und die sich in der Höhe treffen, die durch einen unteren Rand des zweiten Teils erreicht werden soll.

5. Kompressionsstrumpf nach einem der vorhergehenden Ansprüche, wobei der erste Teil aus einer geschlossenen Zehenform besteht.

6. Kompressionsstrumpf nach einem der vorhergehenden Ansprüche, wobei der erste Überlappungsabschnitt (24) einen unteren Rand aufweist, der in Verwendung über dem Fußknöchel liegt.

7. Kompressionsstrumpf nach Anspruch 6, wobei der untere Rand des ersten Überlappungsabschnitts (24) in Verwendung zwischen 5 und 10 Zentimeter über dem Spitzenpunkt des Innenfußknöchels liegt.

8. Kompressionsstrumpf nach einem der vorhergehenden Ansprüche, wobei der untere Rand des ersten Überlappungsabschnitts (24) in Verwendung zwischen 6 und 8 Zentimeter über dem Spitzenpunkt des Innenfußknöchels liegt.

9. Kompressionsstrumpf nach einem der vorhergehenden Ansprüche, wobei, in Verwendung, der erste und der zweite Überlappungsabschnitt (24, 32) im Wesentlichen gleiche Drücke beitragen.

10. Kompressionsstrumpf nach einem der vorhergehenden Ansprüche, wobei der zweite Überlappungsabschnitt (32) in einer Richtung, die sich in Verwendung entlang des Beins erstreckt, tiefer als der erste Überlappungsabschnitt (24) ist.

11. Kompressionsstrumpf nach Anspruch 10, wobei der zweite Überlappungsabschnitt (32) zwischen 0,5 Zentimeter und 1,5 Zentimeter tiefer als der erste Überlappungsabschnitt (24) ist.

12. Kompressionsstrumpf nach einem der vorhergehenden Ansprüche, wobei ein Bereich des Fußabschnitts (16) in der Nähe der Achillesferse in Verwendung ausgebildet ist, um eine geringere elastische Steifigkeit als das ihn umgebende Material aufzuweisen und sich so während einer Dorsalflexion bevorzugt zu dehnen.

13. Verfahren zum Anziehen eines Kompressionsstrumpfes (10), umfassend das Ausbilden des Kompressionsstrumpfes in wenigstens zwei Teile, wobei ein erster Teil (12) des Kompressionsstrumpfes Folgendes umfasst:
einen Fußabschnitt (16), der konfiguriert ist, um an dem Fuß getragen zu werden;
einen Knöchelabschnitt (18), der konfiguriert ist, um sich in Verwendung über dem Fußknöchel zu erstrecken;
einen ersten Überlappungsabschnitt (24) über dem Knöchelabschnitt, wobei der erste Überlappungsabschnitt (24) ausgebildet ist, um in Verwendung einen Druck auszuüben, der im Vergleich zu einem durch den Knöchelabschnitt (18) ausgeübten Druck reduziert ist; und
eine sichtbare äußere Markierung;
und wobei ein zweiter Teil (30) des Kompressionsstrumpfes röhrenförmig ist und Folgendes umfasst:
einen zweiten Überlappungsabschnitt (32) und
einen Beinabschnitt (34), der sich in Verwendung aus dem Überlappungsabschnitt an dem Bein hinauf erstreckt, um wenigstens einen Teil des Unterschenkels zu umgeben,
wobei das Verfahren **dadurch gekennzeichnet ist, dass** es das Anziehen des ersten Teils (12) zuerst und anschließendes Ziehen des zweiten Teils (30) über den ersten Teil und das Hochziehen an dem Bein hinauf umfasst, um den zweiten Überlappungsabschnitt (32) um den ersten Überlappungsabschnitt (24) herum in Position zu bringen, so dass der Durchgang des zweiten Teils (30) über dem Fuß und dem Knöchel durch den ersten Teil vereinfacht wird,
und dadurch, dass die sichtbare äußere Markierung derart gelegen ist, dass sie aufgedeckt wird, wenn der zweite Teil weit genug an dem Bein hinauf hochgezogen wurde, um den ersten und den zweiten Überlappungsabschnitt in ihre vorgesehene Position zueinander zu bringen.

14. Verfahren nach Anspruch 12, ferner umfassend das Ausbilden des ersten und des zweiten Teils (12, 30) in einer derartigen Weise, dass die Reibung zwischen ihnen beim Ziehen des zweiten Teils (30) über den ersten Teil gering genug ist, um das Ziehen des zweiten Teils (30) über den ersten Teil (12) zu ermöglichen, ohne dass der erste Teil (12) entlang des Schenkels gezogen wird.

## Revendications

1. Bas de contention (10) comprenant une première partie (12) comprenant :
une partie pied (16) conçue pour être portée sur le pied ;
une partie cheville (18) conçue pour s'étendre en cours d'utilisation au-dessus de la malléole ; et
une première partie de chevauchement (24) au-dessus de la partie cheville (18), la première partie de chevauchement (24) étant formée pour exercer en cours d'utilisation une pression qui est réduite par rapport à une pression exercée par la partie cheville (18),
et une seconde partie (30) qui est tubulaire et comprend
une seconde partie de chevauchement (32) et
une partie jambe (34) qui s'étend en cours d'utilisation vers le haut de la jambe depuis la partie de chevauchement pour entourer au moins une partie de la jambe,
le bas de contention étant **caractérisé en ce que** la seconde partie de chevauchement (32) peut être placée autour de la première partie de chevauchement (24), le bas de contention fournissant un profil de pression gradué,
**en ce qu'**une surface externe de la première partie (12) et une surface interne de la seconde partie (30) sont formées de manière à fournir un faible coefficient de frottement entre elles, permettant l'enfilage du bas de contention en enfilant d'abord la première partie (12), puis en tirant la seconde partie (30) sur la première partie (12) et en la tirant vers le haut de la jambe pour amener la seconde partie de chevauchement (32) en position autour de la première partie de chevauchement (34), de telle sorte que le passage de la seconde partie sur le pied et la cheville est facilité par la première partie,
et **en ce que** la première partie (12) est dotée d'un marquage extérieur visible (60) dont l'exposition indique quand la seconde partie a été tirée jusqu'à sa position souhaitée.

2. Bas de contention selon la revendication 1, fourni avec un ensemble d'instructions pour un utilisateur, indiquant à l'utilisateur d'enfiler le bas en enfilant d'abord la première partie (12), puis en tirant la seconde partie (30) sur la première partie (12).

3. Bas de contention selon l'une quelconque des revendications précédentes, dans lequel le marquage (60) est disposé en dessous de la première partie de chevauchement (24) et a une certaine étendue verticale, le marquage (60) changeant de manière progressive sur cette étendue verticale de telle sorte que lorsque la seconde partie (30) est tirée vers le haut de la jambe, la partie exposée du marquage procure une base pour juger de combien la seconde partie (30) doit encore être tirée afin d'atteindre sa position requise.

4. Bas de contention selon la revendication 3, dans lequel le marquage (60) comprend une paire de lignes ou de bords (62, 64) qui convergent vers le haut, et qui se rejoignent au niveau devant être atteint par un bord inférieur de la seconde partie.

5. Bas de contention selon l'une quelconque des revendications précédentes, dans lequel la première partie est en forme de pointe fermée.

6. Bas de contention selon l'une quelconque des revendications précédentes, dans lequel la première partie de chevauchement (24) a un bord inférieur qui, en cours d'utilisation, est au-dessus de la malléole.

7. Bas de contention selon la revendication 6, dans lequel le bord inférieur de la première partie de chevauchement (24) se situe, en cours d'utilisation, entre 5 et 10 centimètres au-dessus du sommet de la malléole médiale.

8. Bas de contention selon l'une quelconque des revendications précédentes, dans lequel le bord inférieur de la première partie de chevauchement (24) se situe, en cours d'utilisation, entre 6 et 8 centimètres au-dessus du sommet de la malléole médiale.

9. Bas de contention selon l'une quelconque des revendications précédentes, dans lequel, lors de l'utilisation, les première et seconde parties de chevauchement (24, 32) apportent des pressions sensiblement égales.

10. Bas de contention selon l'une quelconque des revendications précédentes, dans lequel la seconde partie de chevauchement (32) est plus profonde, dans une direction s'étendant, en cours d'utilisation, le long de la jambe, que la première partie de chevauchement (24).

11. Bas de contention selon la revendication 10, dans lequel la seconde partie de chevauchement (32) est plus profonde à raison de 0,5 centimètre à 1,5 centimètres que la première partie de chevauchement (24).

12. Bas de contention selon l'une quelconque des revendications précédentes, dans lequel une zone de la partie pied (16) à proximité du talon d'Achille en cours d'utilisation est formée de manière à avoir une rigidité élastique inférieure à celle du matériau qui l'entoure, et s'étirer ainsi préférentiellement pendant une dorsiflexion.

13. Procédé d'enfilage d'un bas de contention (10), comprenant la formation du bas de contention en au moins deux parties, une première partie (12) du bas de contention comprenant
une partie pied (16) conçue pour être portée sur le pied ;
une partie cheville (18) conçue pour s'étendre en cours d'utilisation au-dessus de la malléole ;
une première partie de chevauchement (24) au-dessus de la partie cheville, la première partie de chevauchement (24) étant formée de manière à exercer en cours d'utilisation une pression qui est réduite par rapport à une pression exercée par la partie cheville (18) ; et
un marquage extérieur visible ;
et une seconde partie (30) du bas de contention étant tubulaire et comprenant
une seconde partie de chevauchement (32) et
une partie jambe (34) qui s'étend, en cours d'utilisation, vers le haut de la jambe depuis la partie de chevauchement pour entourer au moins une partie de la jambe,
le procédé étant **caractérisé en ce qu'**il comprend l'enfilage de la première partie (12) en premier, puis la tension de la seconde partie (30) sur la première partie et son tirage vers le haut de la jambe pour amener la seconde partie de chevauchement (32) en position autour de la première partie de chevauchement (24), de telle sorte que le passage de la seconde partie (30) sur le pied et la cheville est facilité par la première partie,
et **en ce que** le marquage extérieur visible est situé de manière à être révélé lorsque la seconde partie a été tirée assez haut sur la jambe pour amener les première et seconde parties de chevauchement dans leur position prévue l'une par rapport à l'autre.

14. Procédé selon la revendication 12, comprenant en outre la formation des première et seconde parties (12, 30) de telle sorte qu'un frottement entre elles lorsque la seconde partie (30) est tirée sur la première partie est suffisamment faible pour permettre à la seconde partie (30) d'être tirée sur la première partie (12) sans tirer la première partie (12) le long de la jambe.
